# EUROPEAN PATENT APPLICATION

(11) **EP 1 690 516 A2**
(43) Date of publication of application: **16.08.2006**
(21) Application number: 06425066.5
(22) Date of filing: 07.02.2006
(51) Int. Cl.: A61F 2/38, A61F 2/46

(54) **Unicompartmental knee prosthesis and equipment for preparing a seat for the prosthesis in the joint surface**

(30) Priority: 15.02.2005 IT MI20050221
(71) Applicant: Permedica S.p.a., 23807 Merate (Lecco) (IT)
(72) Inventor: Pasquale, Leonardo, 23807 Merate (Lecco) (IT)
(74) Representative: Leihkauf, Steffen Falk

(57) **Abstract**

A unicompartmental knee prosthesis comprises a plate (5) with a sliding surface (6) intended to face a corresponding joint surface (4) and an anchoring surface (8) intended to engage a bottom surface (10) of a bone seat (3), as well as a peripheral edge (11) facing a peripheral wall (12) of the bone seat (3), wherein peripheral anchoring means (14, 15, 16, 17, 18, 23, 24, 26) are provided arranged at the peripheral edge (11) and suitable to engage the peripheral wall (12) of the bone seat (3) such that an immediate connection is provided between the peripheral wall (12) of the bone seat (3) and the peripheral edge (11) of the prosthesis.

## Description

The present invention relates to a unicompartmental knee prosthesis that can be fixed in a seat previously made in the joint surface of the femur and/or tibia.

It is known that, in many cases, a total prosthesis replacing the entire knee is not required in order to reconstruct a damaged knee, therefore partial or unicompartmental prostheses have been developed which only replace one of the two medial and lateral joint surfaces of the knee. Due to the small size of the partial prostheses relative to the total ones, the partial prostheses can be implanted through smaller operative apertures than required for implanting a total prosthesis.

The seats accommodating the partial prostheses, particularly the seat for a tibial prosthesis, are known to be made by milling the bone structure by means of a ball mill, such as a so-called arthroscopic burr. The mill is guided along a line previously drawn on the tibial plate to carry out a first milling in the form of an annular slot. Subsequently, all the bone material within the annular slot is removed by means of milling, such that a seat is obtained having the same shape and size as the prosthesis to be implanted. Such processing is usually long and not always sufficiently accurate, as it lacks any fixed geometrical reference.

To prepare a seat for a femoral prosthesis, swing saws are normally used which have such a size as to prevent that the bone seat is processed through the arthroscopic route.

By means of the known systems, the seat is very difficult to be made with such a shape and depth that are sufficiently accurate to ensure that the prosthesis is stably and durably fixed.

The poor precision of the prior art tools together with the impossibility of carrying out the preparation of the prosthesis seat through the arthroscopic route make the application of knee prostheses difficult for the surgeon and often very uncomfortable for the patient.

Furthermore, when the seat and the prosthesis are not perfectly complementary, the latter tends to swing within the seat, thereby the connection with the surrounding bone tissue is more and more loosened. To solve this problem, high amounts of bone cement are normally used to balance the size tolerances of the seat relative to the prosthesis, the prosthesis being as well anchored by means of screws or pegs.

The object of the present invention is thus to provide a unicompartmental prosthesis for a knee and an equipment for preparing a seat for the prosthesis in the joint surface of the knee, having such characteristics as to solve the drawbacks mentioned with reference to the prior art.

A particular object of the present invention is that it provides a prosthesis promoting a strong connection between the bone seat and the prosthesis as well as a suitable tool for providing, through the arthroscopic route, a bone seat that is particularly suited for accommodating the prosthesis according to the invention.

This object is achieved by means of a unicompartmental prosthesis for a knee that can be fixed in a bone seat made in a joint surface of the knee, in which the prosthesis comprises a plate having:
- a sliding surface formed on a proximal side of the plate and intended to face a corresponding joint surface opposite the bone seat;
- an anchoring surface formed on a distal side of the plate opposite the proximal side, said anchoring surface being intended to engage a bottom surface of the bone seat;
- a peripheral edge arranged between the distal side and the proximal side of the plate and intended to face a peripheral wall of the bone seat,
characterized in that it comprises peripheral anchoring means arranged at the peripheral edge and suitable to engage the peripheral wall of the bone seat such that an immediate connection is provided between the peripheral wall of the bone seat and the peripheral edge of the prosthesis.

Due to this peripheral connection, the prosthesis is positioned relative to the bone seat in an immediate and reliable manner and allows an effective bone ingrowth between the seat and the prosthesis in the days subsequent to the operation.

The object of the present invention is further achieved by means of a mill for preparing a seat in a joint surface to accommodate a prosthesis, in which the mill comprises a working portion and a drive shaft for rotating the working portion about a reference axis R, in which the working portion comprises at least two sharp edges arranged preferably diametrally opposed relative to the reference axis R, such as to allow a circular seat concentric to the reference axis R to be milled, characterized in that the working portion and the drive shaft are two separate pieces having such a shape that they can be inserted through the arthroscopic route in an operative space at said joint surface, in which means are provided for rotatably integrally connecting the working portion to the drive shaft within the operative space.

In order to better understand the invention and appreciate the advantages of the same, several embodiments thereof will be described below by way of non-limiting examples, with reference to the annexed figures, in which:
- Fig. 1 is a perspective view of a tibial prosthesis according to an embodiment of the invention;
- Fig. 2 is a perspective view of a femoral prosthesis according to an embodiment of the invention;
- Fig. 3 is a perspective view of a mill according to an embodiment of the invention;
- Fig. 4 is a perspective view of a tibial prosthesis according to a further embodiment of the invention;
- Fig. 5 is a perspective view of a femoral prosthesis according to a further embodiment of the invention;
- Fig. 6 is a perspective view of a mill according to a further embodiment of the invention;
   Fig. 7A is a schematic sectional view of a detail of a bone seat;
   Fig. 7B is a sectional view of a detail of a prosthesis according to an embodiment;
   Fig. 7C is a partial proximal view of the prosthesis from Fig. 7B.
   Fig. 8A is a schematic sectional view of a detail of a further bone seat;
   Fig. 8B is a sectional view of a detail of a prosthesis according to a further embodiment;
   Fig. 8C is a partial proximal view of the prosthesis from Fig. 8B;
   Fig. 9A is a schematic sectional view of a detail of a further bone seat;
   Fig. 9B is a sectional view of a detail of a prosthesis according to a further embodiment;
   Fig. 9C is a partial proximal view of the prosthesis from Fig. 9B;
   Fig. 10A is a schematic sectional view of a detail of a further bone seat;
   Fig. 10B is a sectional view of a detail of a prosthesis according to a further embodiment;
   Fig. 10C is a partial proximal view of the prosthesis from Fig. 10B;
   Fig. 10D is a schematic side view of the prosthesis from Fig. 10C;
   Fig. 11A is a schematic sectional view of a detail of a further bone seat;
   Fig. 11B is a sectional view of a detail of a prosthesis according to a further embodiment;
   Fig. 11C is a partial proximal view of the prosthesis from Fig. 11B.
   Fig. 12A is a schematic sectional view of a detail of a further bone seat;
   Fig. 12B is a sectional view of a detail of a prosthesis according to a further embodiment;
   Fig. 12C is a partial proximal view of the prosthesis from Fig. 12B.
   Fig. 13A is a schematic sectional view of a detail of a further bone seat;
   Fig. 13B is a sectional view of a detail of a prosthesis according to a further embodiment;
   Fig. 13C is a partial proximal view of the prosthesis from Fig. 13B.
   Fig. 14A is a schematic sectional view of a detail of a further bone seat;
   Fig. 14B is a sectional view of a detail of a prosthesis according to a further embodiment;
   Fig. 14C is a partial proximal view of the prosthesis from Fig. 14B.
   Fig. 15A is a schematic sectional view of a detail of a further bone seat;
   Fig. 15B is a sectional view of a detail of a prosthesis according to a further embodiment;
   Fig. 15C is a partial proximal view of the prosthesis from Fig. 15B;
   Fig. 15D is a schematic side view of the prosthesis from Fig. 15C;
   Fig. 15E is a schematic proximal view of a detail (fitting ring) in Fig. 15A;
   Fig. 16A is a schematic sectional view of a detail of a further bone seat;
   Fig. 16B is a sectional view of a detail of a prosthesis according to a further embodiment;
   Fig. 16C is a partial proximal view of the prosthesis from Fig. 16B;
   Fig. 16D is a schematic side view of the prosthesis from Fig. 16C;
   Fig. 16E is a schematic proximal view of a detail (fitting ring) from Fig. 16A;
   Fig. 17 is a side view of the mill from Fig. 6;
   Fig. 18 is a top view of the mill from Fig. 6;
   Fig. 19 and 20 are further perspective views of the mill from Fig. 6;

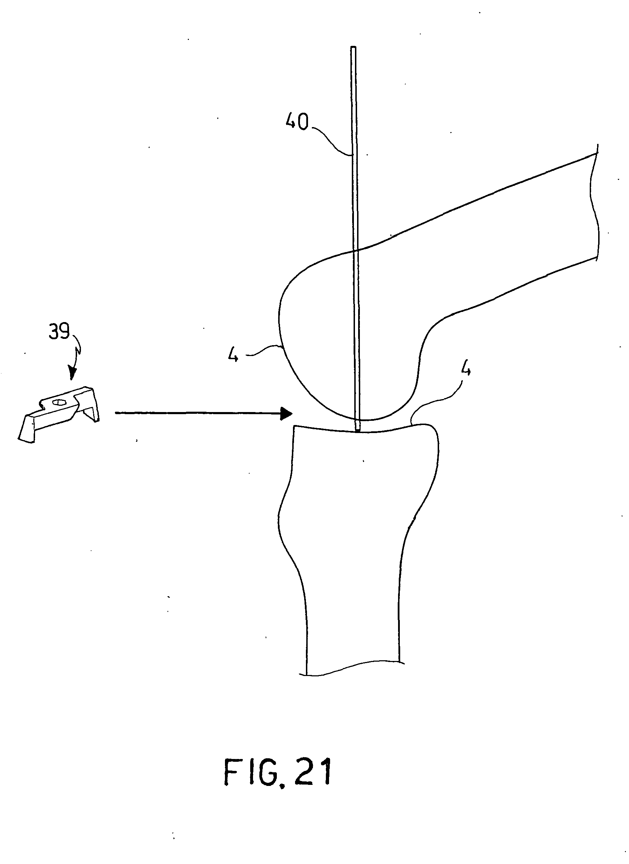

   Fig. 21 is a schematic illustration of an operation to a knee;
   Fig. 22 and 23 show joint surfaces of the knee with prosthesis seats according to the invention;

With reference to the figures, a unicompartmental prosthesis for a knee comprises a tibial prosthesis 1 and/or a femoral prosthesis 2 to be fixed in a bone seat 3 provided in a joint surface 4 of the knee. The prosthesis comprises a plate 5 with a sliding surface 6 formed on a proximal side 7 of the plate 5 and intended to face a corresponding joint surface opposite the bone seat 3, as well as an anchoring surface 8 formed on a distal side 9 of the plate 5 opposite the proximal side 7. The anchoring surface 8 is intended to engage a bottom surface 10 of the bone seat 3;

The plate 5 further comprises a peripheral edge 11 arranged between its distal 9 and proximal 7 sides and intended to face a peripheral wall 12 of the bone seat 3.

At the peripheral edge 11 there are provided peripheral anchoring means suitable to engage the peripheral wall 12 of the bone seat 3 such that an immediate connection is provided between the peripheral wall 12 and the peripheral edge 11 of the prosthesis.

In accordance with an embodiment, said peripheral anchoring means are embodied by a surface roughness or rough coating of the surface of the peripheral edge 11, such that high friction is provided between the peripheral wall 12 and the peripheral edge 11 of the prosthesis.

Advantageously, the peripheral anchoring means comprise barb means and/or sharp means and/or threaded means and/or expansion means that will be described in greater detail below.

The peripheral anchoring means preferably comprise projections protruding from the peripheral edge 11 and are suitable to engage suitable cavities 13 in the peripheral wall 12 of the bone seat 3.

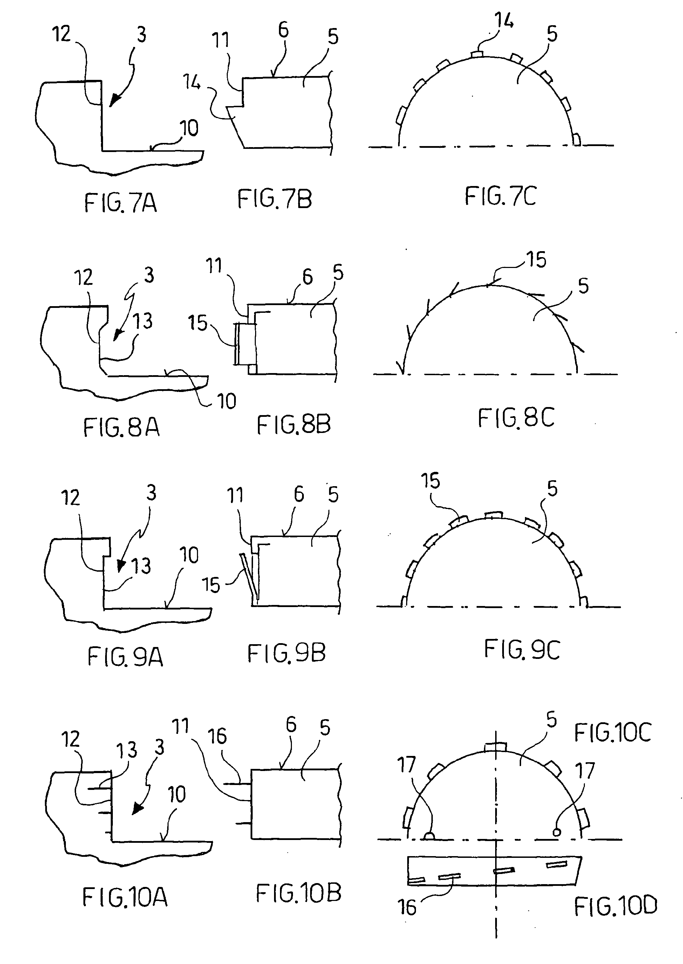

In accordance with an embodiment, the projections are suitable to produce, for example to cut out, said cavities 13 in the peripheral wall 12 of the bone seat 3 when the prosthesis is being inserted into the bone seat 3 (Fig. 7 and 10).

The projections comprise, for example, one or more circumferential ribs (not shown in the figures) or a plurality of teeth 14 arranged along the peripheral edge 11 of the plate 5 and preferably biased such that the prosthesis can be inserted by means of impacting.into the bone seat 3 and prevented from escaping therefrom (Fig. 7) .

In accordance with a further embodiment, the projections comprise tongues 15 that are formed from the peripheral edge 11 and radially folded outwards. These tongues 15 can be folded about a transversal or biased axis relative to the plane of the plate 5 (Fig. 8B and 8C) or they are folded about an axis parallel to the plane of the plate 5 (Fig. 9B and 9C).

In the embodiment shown in the Fig. 10A to 10D, the projections comprise one or more sharp edges or tabs 16 helically arranged about the peripheral edge 11 of the plate 5, such that a self-tapping threading is formed. In this case, the plate 5 comprises suitable gripping means, such as two spaced apart seats 17 formed in the sliding surface 6, which allow inserting a tool for screwing the plate 5 within the bone seat 3.

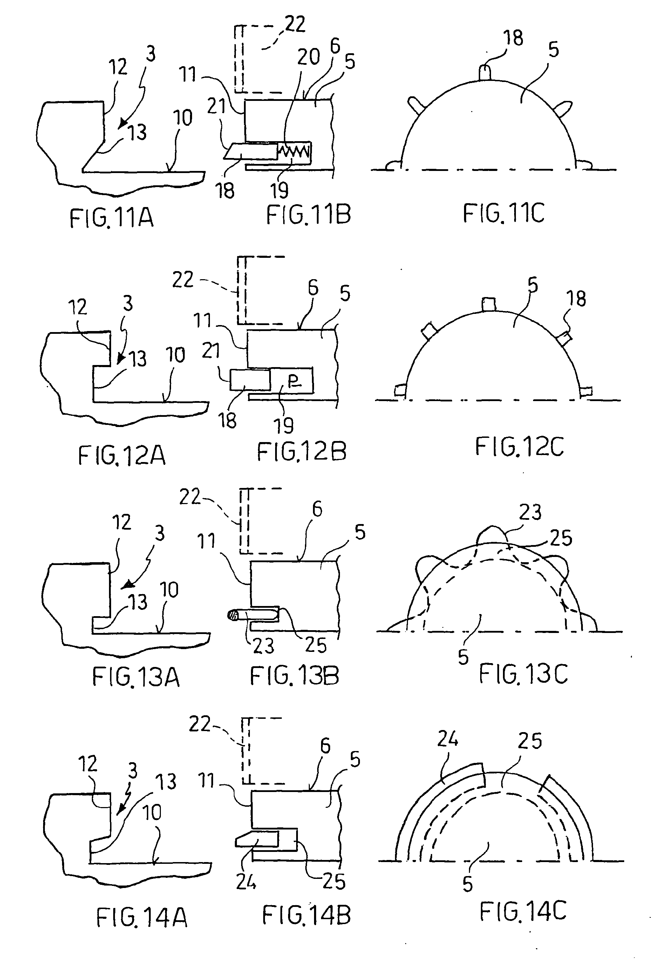

In accordance with a further embodiment (Fig. 11A to 12C), the peripheral anchoring means comprise one or more latch bodies, such as pins 18, which are accommodated in suitable seats 19 provided in the peripheral edge 11 of the plate 5.

Biasing means, such as preloaded springs 20 or pressurized fluids P that are advantageously arranged in said seats 19 (the prosthesis is assembled for example in a high pressure environment) permanently bias the latch body in an engaging position, where at least one end 21 of the latch body protrudes from the peripheral edge 11 of plate 5.

A retainer ring 13 (outlined in Fig. 11B, 12B, 13B, 14B) is provided for maintaining the latch bodies in a withdrawn position within the seats 19 until the plate 5 is inserted in the bone seat 3.

According to a further embodiment (Fig. 13 and 14), the latch body is embodied by an elastic ring 23 or ring segment 24 accommodated in a circumferential seat 25 provided in the peripheral edge 11 of plate 5. The ring 23 or ring segment 24 can be pressed, due to a suitable retainer ring 22, in a withdrawn position within the circumferential seat 25 and tends, in the absence of the compression force exerted by the retainer ring 22, to expand to an engaging position, where it partially protrudes from said circumferential seat 25.

According to the embodiment shown in Fig. 13B and 13C, the elastic ring 23 is a preferably metallic ring having an undulated shape.

According to the embodiment shown in Fig. 14B and 14C, the ring segment 24 is a flat and curve, preferably metallic, profile of the "GLOWER" type.

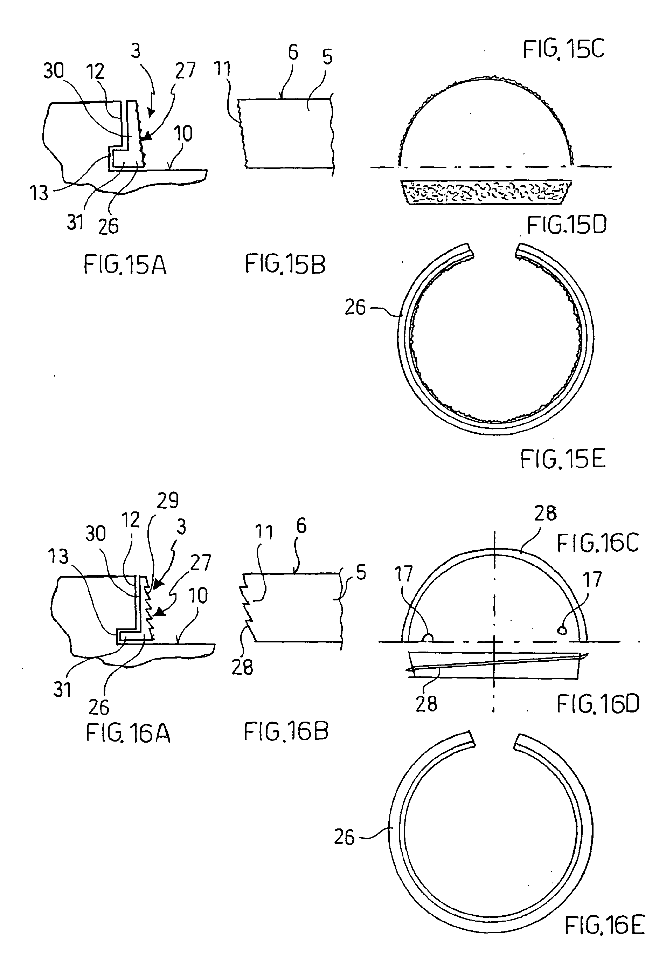

In accordance with a yet further embodiment of the invention (Fig. 15 and 16), the prosthesis, i.e. the peripheral anchoring means comprise a fitting ring 26 to be positioned within the bone seat 3. The fitting ring 26 defines an artificial seat 27 to house at least the peripheral edge 11 of the plate 5, in which the peripheral edge 11 and the artificial seat 27 have such a shape that when the plate 5 is inserted within the fitting ring 26 arranged within the bone seat 3 the fitting ring 26 is expanded. Due to this expansion, an interference or shape connection is obtained between the fitting ring 26 and the peripheral wall 12 of the bone seat 3 and between the artificial seat 27 of the fitting ring 26 and the peripheral edge 11 of the plate 5.

Preferably, the artificial seat 27 of the fitting ring 26 and/or the peripheral edge 11 of the plate 5 are substantially shaped as a truncated cone.

In order to prevent that the plate 5 may escape from the artificial seat 27, the latter comprises one or more ribs or steps or roughness suitable to engage mating ribs or steps or roughness formed in the peripheral edge 11 of the plate 5 (Fig. 15A and 15B).

According to the embodiment shown in the figures 16A to 16E, the peripheral edge 11 of the plate 5 comprises a threading 28 suitable to engage a mating threading 29 formed within the artificial seat 27 of the fitting ring 26. In this case, the plate 5 comprises suitable gripping means, such as two spaced apart seats 17 for allowing the plate 5 to be screwed within the artificial seat 27.

Advantageously, one of the threadings 28, 29, preferably the one of the plate 5, is self-tapping and suitable to form the other threadings when the plate 5 is being screwed into the fitting ring 26.

The fitting ring 26 comprises, in turn, a circumferential outer edge 30 intended to engage the peripheral wall 12 of the bone seat 3 and circumferential anchoring means arranged at the circumferential outer edge 30 and suitable to engage the peripheral wall 12 of the bone seat 3 such that said interference or shape connection is provided.

The circumferential anchoring means preferably comprise one or more projections 31 or steps or a threading or roughness or means analogue to the peripheral anchoring means of the plate 5 described above.

Advantageously, with the tibial prosthesis 2, the plate 5 is substantially circular with a diameter ranging from 15 mm to 35 mm, preferably 20 mm to 30 mm.

With the femoral prosthesis 2, the plate 5 has a preferably elongated and curved shape with a length ranging from 25 mm to 45 mm and a width ranging from 10 mm to 20mm.

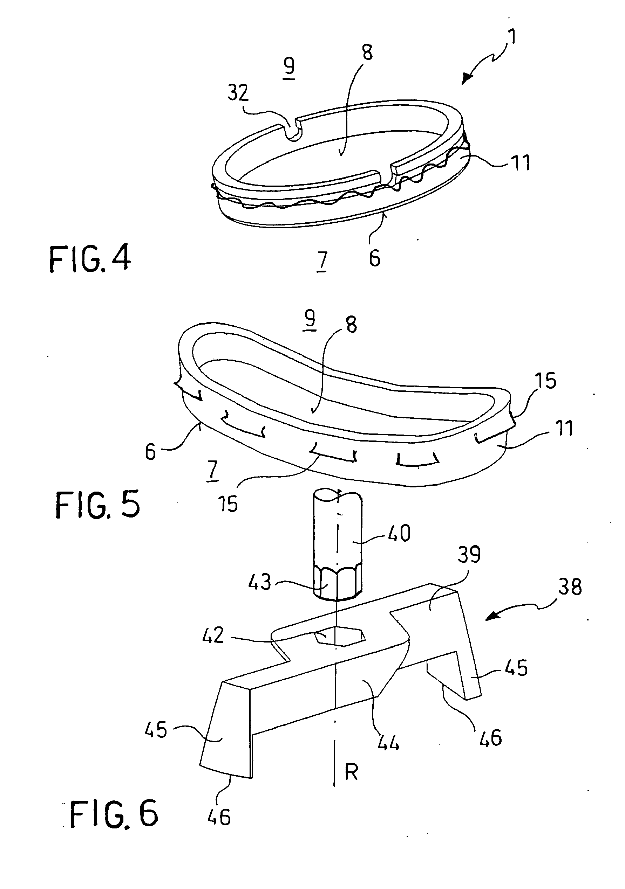

In accordance with an embodiment, such as shown in Fig. 4, the peripheral edge 11 defines one or more holes or cavities 32 radially passing through the peripheral edge 11 and allow to gain access to the space between the peripheral edge 11 and the bone seat 3 from the outside such that a surgical instrument, or when required, bone cement can be inserted.

It should be noted herein that, due to the immediate peripheral coupling, the prosthesis is firmly positioned within the bone seat, thereby a quick and effective bone ingrowth is favoured and the use of cement for further connecting the prosthesis to the surrounding spongiosa is mostly avoided.

Advantageously, the anchoring surface 8 of the plate 5 has a rough layer, preferably obtained by a coating of powder of titanium or titanium dioxide which favours bone ingrowth.

The tibial prosthesis 1 and/or femoral prosthesis 2 are preferably made of high resistant polyethylene or a cobalt-chromium alloy or steel alloy.

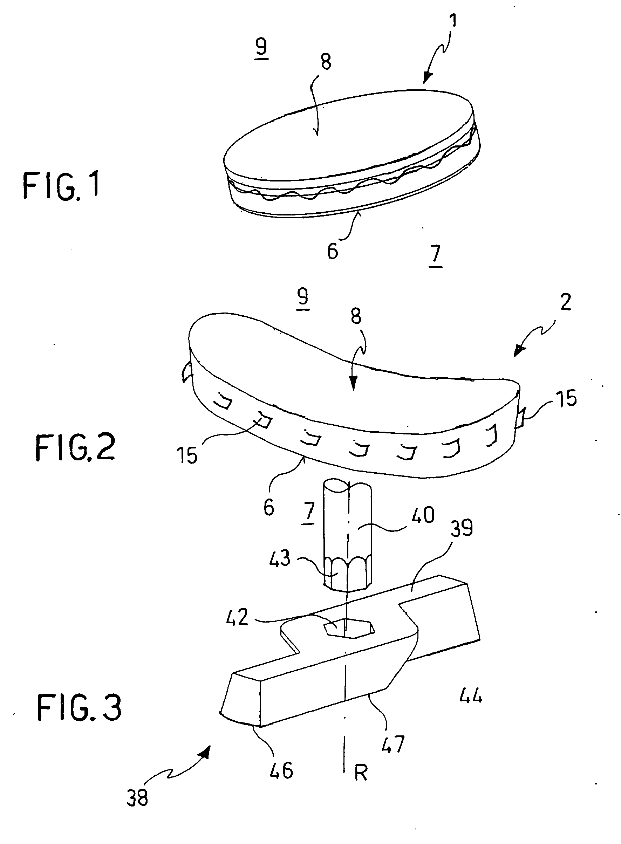

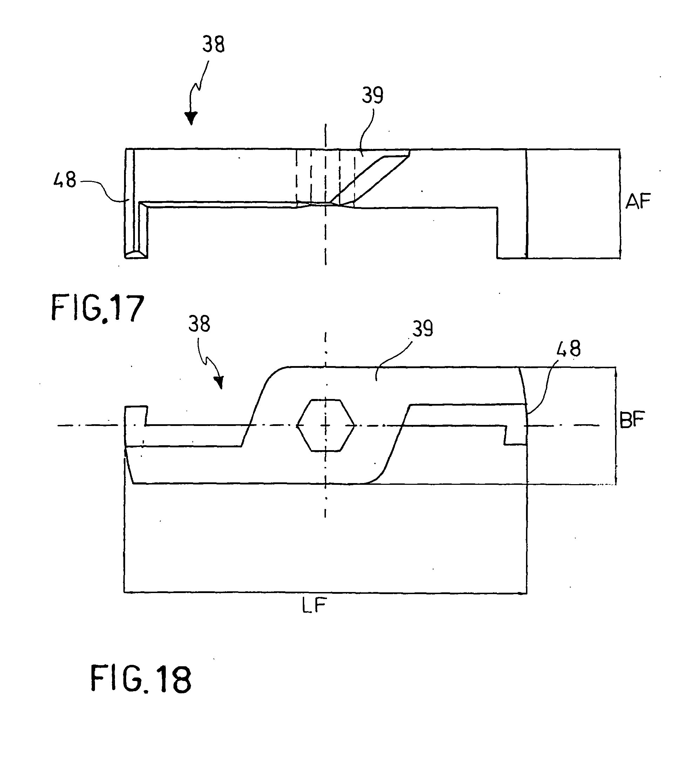

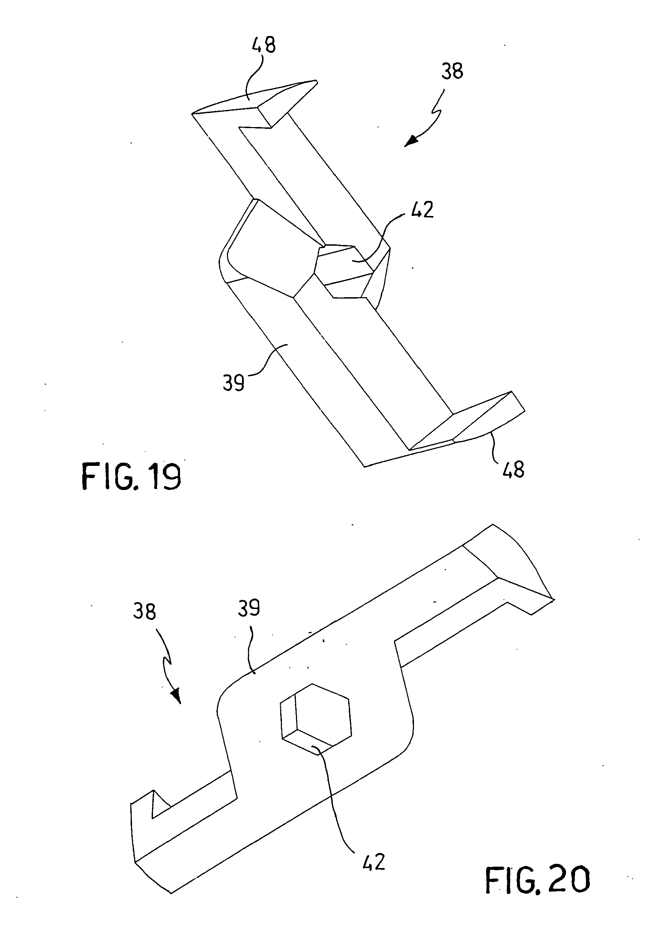

To provide said bone seat 3 in the joint surface, particularly to provide a perfectly circular seat, a suitable mill 38 has been developed which will be described below with reference to Fig. 3, 6, 17, 18, 19 and 20.

The mill 38 comprises a working portion 39 and a drive shaft 40, either rigid or flexible, suitable to rotate the working portion 39 about a reference axis R. The working portion 39 comprises one or more sharp edges 46, 47 radially extending outside the reference axis R, such as to allow milling a circular bone seat concentric to the reference axis R. The working portion 39 and the drive shaft 40 are two separate pieces and each of them has a shape such as to be introduced through the arthroscopic route in an operative space at the joint surface 4. The mill further comprises suitable means for rotatably integrally and removably connecting the working portion 39 to the drive shaft 40 inside the operative space.

Advantageously, the connecting means comprise a polygonal, preferably hexagonal, seat 42 provided within the working portion 39 and suitable to accommodate a corresponding polygonal, preferably hexagonal, portion 43 of the drive shaft 40.

The working portion 39 comprises a middle portion 44 defining said hexagonal seat 42 and two outer working portions 45 with sharp edges 46.

In accordance with the embodiment as shown in Fig. 6, the outer working portions 45 (i.e. the sharp edges 46) project relative to the middle portion 44 in a working direction and are arranged diametrally opposite to each other. With this configuration, when the mill 38 is being rotated about the reference axis R, the two outer working portions 45 define a rotational figure (Fig. 6) having the shape of a circular ring which protrudes from the plane in which the middle portion 44 rotates or having the shape of a circular disc (Fig. 3) to be able to mill the bone seat 3 having the shape of a circular ring or circular disc concentric to the reference axis R.

In accordance with a further embodiment, the middle portion 44 also comprises one or more middle sharp edges 47 facing the working direction and arranged such that, when the mill 38 is rotated about the reference axis R, the middle sharp edges 47 define a substantially planar rotating figure which is withdrawn relative to the outer working portions (23). This allows surface-grinding the bottom surface 10 of the bone seat simultaneously with milling a deeper peripheral area thereof in the shape of a circular ring.

Advantageously, the whole working portion 39 of the mill 38 is provided as one piece in a metallic material, preferably stainless steel.

In accordance with the preferred embodiment, the working portion 39 has the shape of an elongated rod such that the mill 38 is more easily inserted, through the arthroscopic route, within the operative space.

In order to provide the bone seat 3 for the tibial prosthesis as described above, the working portion 39 of the mill 38 has a length LF ranging from 15 mm to 35 mm, similar to the diameter of the tibial prosthesis, a width BF ranging from 6 mm to 8 mm and a height AF ranging from 4 mm to 8 mm.

The outer working portions 45 have a radially outer surface 48, which is rounded as an arc of circle having the length of the mill working portion 39 as the diameter.

A method for implanting the prosthesis according to the invention with the aid of the mill according to the invention will be described below.

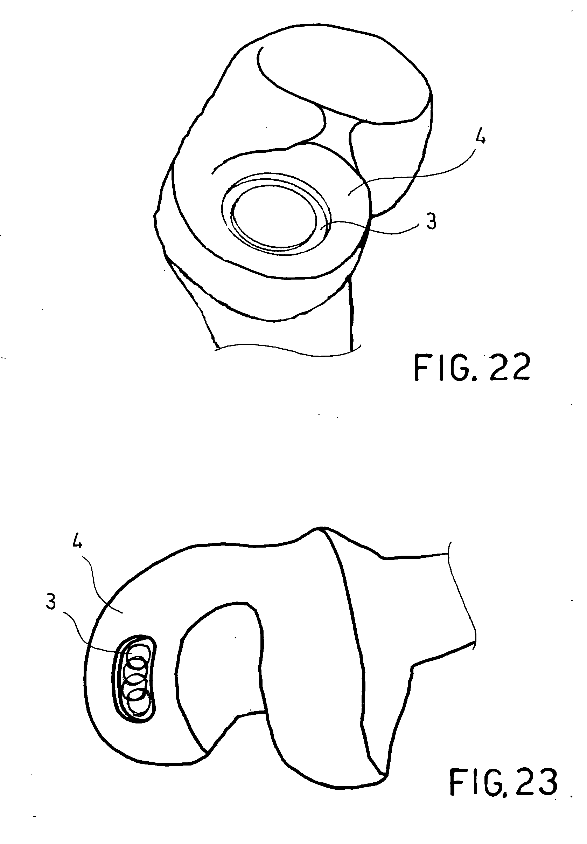

After a short exploratory arthroscopy of the knee, the tibial surface is measured by means of graduated needles and the position is established where the tibial milling hole is to be drilled. Through the transfemoral route, at about 60° knee flexion, a Kirschner wire 27 is applied, such as 1,6 mm diameter, by means of a suitable guide centered on the milling spot previously established on the tibia (see Fig. 12). When the Kirschner wire emerges, the mill 20 is introduced through the arthroscopic route or however through a reduced operating aperture. The femur is then drilled with a cannulated tip such as 3,2 mm along the axis of the Kirschner wire, the cannulated tip is extracted and the cannulated drive shaft is applied with the hexagonal end. The hexagonal end of the mill-holder shaft is joined to the hexagonal seat of the mill, the mill is positioned and caused to rotate in a first direction (such as counter-clockwise) until a perfect rotation circle is obtained about the reference axis R which further stabilizes the mill. The rotation of the mill is reversed by turning the same in a cutting direction (such as clockwise) until the mill is dipped into the tibial plate to obtain a perfectly circular bone seat as may be seen for example in Fig. 22.

An arthroscopic washing of the bone fragments is then carried out.

To provide an elongated seat, such as in the femoral joint surface, a line is drawn along this joint surface corresponding to the load point of the femur and, for example, 4 or 5 drillings about 7 mm away from each other are performed by means of a Kirschner wire along this line. Thereafter, processing is carried out by means of the mill having a diameter equal to the width of the femoral prosthesis, centered on the Kirschner wire for each of the drillings such that a working is obtained in the shape of cross rings, such as schematically illustrated in Fig. 23. When required, the obtained seat shape can be adjusted by means of a small chisel. An arthroscopic washing of the bone fragments is then carried out.

The prosthesis according to the invention is applied by widening the arthroscopic incision to about 2 cm and inserting the tibial and/or femoral prosthesis within the knee, positioning the same at the bone seat thereof and applying the same, such as by means of impact, using a suitable impactor and employing a tab of plastic material to protect the sliding surface of the prosthesis.

The prosthesis and the mill according to the present invention have a number of advantages.

Due to the immediate anchoring effect due to the engagement of the peripheral anchoring means with the peripheral wall of the bone seat, a stable positioning of the prosthesis is obtained, which favours an effective bone ingrowth in the post-surgery days without using invasive clamping means such as screws or pegs.

The prosthesis (due to its planar shape and the absence of portions protruding from the plate plane) can be applied through a very narrow operative access, and in some case, through the arthroscopic route. Due to the immediate peripheral anchorage, the prosthesis according to the invention obviates to the use of cement (such as polymethylmethacrylate), reinforcement keels and the so-called anchoring "pegs" used in the prior art prostheses.

The mill according to the invention allows the bone seat to be fully and exclusively worked by means of arthroscopy, thereby simultaneously increasing the accuracy of the same.

The peripheral anchorage is mechanically very stable and dramatically reduces the swinging effects of the prosthesis as well as the effects of the tangential forces on the femoral prosthesis.

Furthermore, the mutual engagement between the peripheral edge of the prosthesis and the peripheral wall of the bone seat is particularly accurate, due to the mill which allows obtaining perfect circular seats. This is particularly advantageous for applying the prosthesis by means of interference connection, such as in a press-fit manner without employing cement.

Moreover, the particular shape of the prosthesis (without screws) and the corresponding complementary shape of the seat that can be obtained due to the particular shape of the mill, imply, in addition to the high anchoring effect, a coating of only the cartilage defect (optionally only grinded on the surface) and not of the entire joint surface (also the healthy one) with a minimum thickness of the plates (of the tibial and femoral prostheses) facing each other.

This also allows reducing the size of the prosthesis only to the extension of the cartilage defect, which makes the same less invasive than the prior art prostheses that are often sized according to the anchoring requirements.

The possibility of reducing the prosthesis size due to the peripheral anchoring system is crucial for unilateral applications of prosthesis inlays only on the tibia or femur when the cartilage of the opposite joint surface is still intact.

## Claims

1. A unicompartmental prosthesis for a knee comprising a tibial prosthesis (1) and/or a femoral prosthesis (2) to be fixed in a bone seat (3) provided in a joint surface (4) of the knee, wherein the prosthesis comprises a plate (5) having:
- a sliding surface (6) formed on a proximal side (7) of the plate (5) and intended to face a corresponding joint surface (4) opposite the bone seat (3);
- an anchoring surface (8) formed on a distal side (9) of the plate (5) opposite the proximal side (7), said anchoring surface (8) being intended to engage a bottom surface (10) of the bone seat (3);
- a peripheral edge (11) arranged between the distal side (9) and the proximal side (7) of the plate (5) and intended to face a peripheral wall (12) of the bone seat (3),
**characterized in that** it comprises peripheral anchoring means (14, 15, 16, 18, 23, 24, 26) arranged at the peripheral edge (11) and suitable to engage the peripheral wall (12) of the bone seat (3) such that an immediate connection is provided between the peripheral wall (12) of the bone seat (3) and the peripheral edge (11) of the prosthesis.

2. The prosthesis according to claim 1, wherein said peripheral anchoring means (14, 15, 16, 18, 23, 24, 26) comprise means selected from the group comprising barb means, cutting means, threaded means, expansion means.

3. The prosthesis according to any preceding claim, wherein said peripheral anchoring means (14, 15, 16, 18, 23, 24, 26) comprise projections (14, 15) protruding from the peripheral edge (11), suitable to engage suitable cavities (13) in the peripheral wall (12).

4. The prosthesis according to claim 3, wherein said projections (14, 15) are suitable to cut said cavities (13) in the peripheral wall (12) of the bone seat (3) while the prosthesis is being inserted within the bone seat (3).

5. The prosthesis according to any preceding claims, wherein said projections (14, 15) comprise one or more circumferential ribs or a plurality of teeth (14) arranged along said peripheral edge (11) that are biased such as to allow the prosthesis to be inserted within the bone seat (3) by means of impact, and preventing the same from escaping thereof.

6. The prosthesis according to any preceding claim, wherein said projections (14, 15) comprise tongues (15) obtained from the peripheral edge (11) and radially bent outwards.

7. The prosthesis according to claim 6, wherein said tabs (15) are bent about a transversal axis relative to the plane of the plate (5).

8. The prosthesis according to claim 6, wherein said tabs (15) are bent about a parallel axis relative to the plane of the plate (5).

9. The prosthesis according to claim 6, wherein said tabs (15) are bent about inclined axes relative to the plane of the plate (5).

10. The prosthesis according to any preceding claim, wherein said projections comprise one or more sharp edges or tabs (16) helically arranged about the peripheral edge (11) of the plate (5) such that a self-tapping threading is formed and wherein the plate (5) comprises suitable gripping means (17) allowing the same to be screwed within the bone seat (3).

11. The prosthesis according to claim 10, wherein in the sliding surface (6) there are formed two spaced apart seats (17) allowing a tool to be inserted for screwing the plate (5) within the bone seat (3).

12. The prosthesis according to any preceding claim, wherein said peripheral anchoring means (14, 15, 16, 18, 23, 24, 26) comprise:
- one or more latch bodies (18) accommodated in suitable seats (19) formed in the peripheral edge (11) and
- biasing means (20, p) biasing the latch body (18) permanently to an engaging position, wherein at least one end (21) of the latch body (18) projects from the peripheral edge (11).

13. The prosthesis according to the preceding claim, comprising a retainer ring (22) suitable to hold the latch bodies (18) in a withdrawn position within the seats (19).

14. The prosthesis according to claim 12 or 13, wherein the biasing means comprise elastic means (20) or pressurized fluids (p).

15. The prosthesis according to claim 12 or 13, wherein said latch body is an elastic ring (23) or ring segment (24) accommodated in a circumferential seat (25) formed in the peripheral edge (11) of the plate (5), wherein said ring or ring segment can be pressed into a withdrawn position within the circumferential seat (25) and tends, in the absence of a retaining force, to expand to an engagement position, where it partially projects from said seat (25).

16. The prosthesis according to any preceding claim, comprising a fitting ring (26) to be positioned within said bone seat (3), wherein said fitting ring (26) defines an artificial seat (27) for housing at least said peripheral edge (11) of the plate (5) and said peripheral edge (11) and said artificial seat (27) have such a shape that when the plate (5) is inserted within the fitting ring (26) arranged within the bone seat (3) the fitting ring (26) is expanded such that an interference or shape connection is provided between the fitting ring (26) and the peripheral wall (12) of the bone seat (3) and between the artificial seat (27) of the fitting ring and the peripheral edge (11) of the plate (5).

17. The prosthesis according to the preceding claim, wherein said artificial seat (27) of the fitting ring (26) and/or the peripheral edge (11) of the plate (5) is substantially shaped as a truncated cone.

18. The prosthesis according to claim 16 or 17, wherein said artificial seat (27) comprises one or more ribs or steps (29) suitable to engage corresponding ribs or steps (28) formed in the peripheral edge (11) of the plate (5) such that the plate (5) is prevented from escaping from the artificial seat.

19. The prosthesis according to claim 16 or 17, wherein said peripheral edge (11) of the plate (5) comprises a threading (28) suitable to engage a mating threading (29) formed in the artificial seat (27) of the fitting ring (26) and wherein the plate (5) comprises suitable gripping means (17) for allowing the same to be screwed within the artificial seat (27).

20. The prosthesis according to the preceding claim, wherein one of the threadings (28; 29) is self-tapping and suitable to form the other threading (29; 28) while the plate (5) is being screwed within the fitting ring (26).

21. The prosthesis according to any claim 16 to 20, wherein the fitting ring (26) comprises a circumferential outer edge (30) suitable to engage the peripheral wall (12) of the bone seat (3) and circumferential anchoring means (31) suitable to engage the peripheral wall (12) of the bone seat (3) such as to provide said interference or shape connection.

22. The prosthesis according to claim 21, wherein said circumferential anchoring means (31) comprise:
- one or more projections (31) or steps or
- a threading or
- a roughness or
- means similar to the peripheral anchoring means (14, 15, 16, 18, 23, 24, 26) of the plate (5) according to any preceding claim.

23. The prosthesis according to any preceding claim, which is a tibial prosthesis (1).

24. The prosthesis according to claim 23, wherein the plate (5) is substantially disc-shaped with a diameter ranging from 15 mm to 35 mm.

25. The prosthesis according to any claim 1 to 22, which is a femoral prosthesis (2).

26. The prosthesis according to claim 25, wherein the plate (5) has an elongated shape having a length ranging from 25 mm to 45 mm and a width ranging from 10 mm to 20 mm.

27. The prosthesis according to any preceding claim, wherein the peripheral edge defines one or more holes or cavities (37) passing through the peripheral edge (11).

28. The prosthesis according to any preceding claim, wherein the anchoring surface (8) of the plate (5) has a rough layer favouring the bone ingrowth.

29. The prosthesis according to claim 28, wherein said rough layer is obtained by means of a coating of titanium oxide or titanium dioxide powder or by means of sandblasting.

30. The prosthesis according to any preceding claim, wherein the plate (5) is made of high resistant polyethylene or a cobalt-chromium alloy.

31. The prosthesis according to any preceding claim, wherein said peripheral anchoring means are embodied by a surface roughness or rough coating of the surface of the peripheral edge (11), such that high friction is provided between the peripheral wall (12) and the peripheral edge (11) of the prosthesis.

32. A mill (38) for preparing a bone seat (3) in a joint surface (4) to accommodate a prosthesis (1, 2), in which the mill (38) comprises a working portion (39) and a drive shaft (40) suitable for rotating the working portion (39) about a reference axis (R), wherein the working portion (39) comprises one or more sharp edges (46, 47) radially extending outside the reference axis (R), such as to allow the milling of a circular bone seat (3) concentric to the reference axis (R), **characterized in that** the working portion (39) and the drive shaft (40) are two separate pieces, each having such a shape that they can be separately inserted through the arthroscopic route in an operative space at the joint surface (4), wherein means (42, 43) are provided for rotatably integrally connecting the working portion (39) to the drive shaft (40) within said operative space.

33. The mill (38) according to claim 32, wherein the working portion (39) comprises a middle portion (44) forming said means (42) for integrally rotatably connecting to the drive shaft (40) two outer working portions (45) with sharp edges (46) projecting relative to the middle portion (44) in a working direction and arranged diametrally opposite to each other, such that when the mill (38) is being rotated about the reference axis (R), the two outer working portions (45) define a rotational figure in the shape of a circular ring or circular disc projecting from the rotational plane of the middle portion (44), such as to allow the milling of said seat (3) in the shape of a circular ring or circular disc concentric with the reference axis (R).

34. The mill (38) according to claim 33, wherein the middle portion (44) comprises one or more middle sharp edges (47) facing the working direction and arranged such that, when the mill(38) is rotating about the reference axis (R), the middle sharp edges (47) define a substantially planar rotational figure, which is withdrawn relative to the outer working portions (45) such as to allow grinding the surface of a bottom surface (10) of the bone seat (3) simultaneously with the milling of a peripheral area of the seat (3) in the shape of a ring by means of the outer working portions (45).

35. The mill (38) according to claim 33 or 34, wherein the working portion (39) is made as one piece from a metallic material, preferably stainless steel.

36. The mill (38) according to any claim 32 to 35, wherein said working portion (39) has the shape of an elongated rod such as to facilitate the insertion of the working portion (39) through the arthroscopic route within the operative space.

37. The mill (38) according to any of claims 32 to 36, wherein the connecting means (42, 43) comprise a polygonal seat (42) formed in the working portion (39) and suitable to accommodate a corresponding polygonal portion (43) of the drive shaft (40).

38. The mill (38) according to any claim 32 to 37, wherein the working portion (39) is 15 mm to 35 mm in length (LF) and 6 mm to 8 mm in width (BF), as well as 4 mm to 8 mm in height (AF).

39. The mill (38) according to any claims 32 to 38, wherein the outer working portions (45) have a radially outer surface relative to the reference axis (R), which is rounded as an arc of circle having the length (LF) of the mill working portion (39) as the diameter.
